# EUROPEAN PATENT SPECIFICATION

(11) **EP 4 200 273 B1**
(45) Date of publication and mention of the grant of the patent: **17.07.2024**
(21) Application number: 21755792.5
(22) Date of filing: 09.08.2021
(51) Int. Cl.: C07C 51/44, C07C 51/235, C07C 53/126, C07C 53/128

(54) **PROCESS FOR THE PREPARATION OF C6-12 SATURATED ALIPHATIC CARBOXYLIC ACIDS**
VERFAHREN ZUR HERSTELLUNG VON C6-12 GESÄTTIGTEN ALIPHATISCHEN CARBONSÄUREN
PROCÉDÉ DE PRÉPARATION D'ACIDES CARBOXYLIQUES ALIPHATIQUES SATURÉS EN C6-12

(30) Priority: 20.08.2020 EP 20191855
(43) Date of publication of application: 28.06.2023
(73) Proprietor: BASF SE, 67056 Ludwigshafen am Rhein (DE)
(72) Inventor: HAMANN, Jessica Nadine, 67056 Ludwigshafen (DE); TELES, Joaquim Henrique, 67056 Ludwigshafen (DE); OHLEMUELLER, Peter Georg, 68623 Lampertheim (DE); GAITZSCH, Friedemann, 67056 Ludwigshafen (DE); LIANG, Shelue, 67056 Ludwigshafen (DE); RITTINGER, Stefan, 68199 Mannheim (DE); THRUN, Frauke, 67056 Ludwigshafen (DE); DEHN, Richard, 67063 Ludwigshafen (DE)
(74) Representative: BASF IP Association
(86) International application number: PCT/EP2021/072122
(87) International publication number: WO 2022/037978

(56) References cited:
- CN-A- 109 438 216
- US-A- 5 504 229

## Description

The present invention relates to a process for the preparation of a saturated aliphatic carboxylic acid with 6 to 12 carbon atoms by oxidation of the corresponding aldehyde with molecular oxygen, in which the saturated aliphatic carboxylic acid is obtained in a high purity with a very low tendency to darken by being tempered under inert gas conditions at a temperature of 225°C and a pressure of 0.1 MPa for 4 hours.

Saturated aliphatic carboxylic acids are important intermediates globally with a wide range of applications. They can be used as such, but are typically further processed to metal salts, esters, amides, anhydrides, acid chlorides, and other derivatives. Overall, they are important intermediates for the production of a wide variety of compounds such as metal salts and metal soaps, flavors, fragrances, pharmaceutical and agrochemical ingredients, cosmetic ingredients, plasticizers, paints, coating additives, coolants, lubricants or catalysts for polymer processing. A very important representative of a C₆₋₁₂ saturated aliphatic carboxylic acid is 2-ethylhexanoic acid. It is mainly used in the form of its derivatives as for example its metal salts or esters for their use as drying agents and thickeners for alkyd resins and paints, as catalysts in polyurethane foam manufacturing, as PVC stabilizers and/or plasticizers, or as anti-wear agents and corrosion inhibitors for lubricants. Esters of C₆₋₁₂ saturated aliphatic carboxylic acids such as of n-heptanoic acid, n-octanoic acid, 2-ethylhexanoic acid, n-nonanoic acid or 3,5,5-trimethylhexanoic acid are also often used as lubricants.

A widely used and important method for the production of saturated aliphatic carboxylic acids with 6 to 12 carbons atoms is the oxidation of the corresponding aldehydes with molecular oxygen in the liquid phase in the presence or absence of a catalyst or any additives. This general synthesis route is, for example, described in J. Kubitschke et al., "Carboxylic acids, aliphatic" in Ullmann's Encyclopedia of Industrial Chemistry, 2014, Wiley-VCH Verlag GmbH & Co. KGaA, DOI: 10.1002/14356007.a05_235.pub2, chapter 4.2.1 "Aldehyde oxidation". The C₆₋₁₂ saturated aliphatic carboxylic acid obtained by the above-mentioned oxidation are then usually purified by distillation to obtain the C₆₋₁₂ saturated aliphatic carboxylic acid in a preferably pure form.

Since most of the applications require a highly transparent and colorless product, not only a high chemical purity of preferably significantly above 99 wt.-% is desired but also a very low APHA color number.

US 5,504,229 describes the preparation of 2-ethylhexanoic acid by oxidation of 2-ethylhexanal in the presence of potassium 2-ethylhexanoate as selectivity improving additive, and its subsequent distillation to obtain purified 2-ethylhexanoic acid at which the potassium 2-ethylhexanoate is enriched in the bottom product and recycled back to the oxidation stage. According to example 1, the obtained 2-ethylhexanoic acid showed a low APHA color number of 4.

CN 109438216 discloses the preparation of 2-ethylhexanoic acid by a multistep production at which n-butyraldehyde was subjected to an aldol condensation to yield 2-ethyl-3-hexenal and subsequently hydrogenated to 2-ethylhexanal. The inventors realized that after the hydrogenation step a small amount of 2-ethyl-3-hexenal remained, which would be oxidized in the oxidation step together with the 2-ethylhexanal causing crude 2-ethylhexanoic acid contaminated with 2-ethyl-3-hexenoic acid, which in turn cannot be separated off from 2-ethylhexanoic acid by distillation. CN 109438216 teaches to transform the 2-ethyl-3-hexenal which remained after the hydrogenation with water in the presence of an acidic catalyst into 2-ethyl-3-hydroxyhexanal which could be oxidized in the oxidation step to 2-ethyl-3-hydroxyhexanoic acid and which in turn could be separated off from the 2-ethylhexanoic acid by distillation. According to examples 1 to 5, 2-ethylhexanoic acid with a purity of up to 99.91 wt.-% and a low APHA color number of up to 3 could be obtained.

It was recognized according to the invention that a low APHA color number of the freshly distilled C₆₋₁₂ saturated aliphatic carboxylic acids is no guarantee that such low APHA color number remains at longer storage nor that it ensures a color stable product for which the respective carboxylic acid was utilized for. It was perceived that the C₆₋₁₂ saturated aliphatic carboxylic acids usually tend to darken over time during storage, when subjected to thermal stress and/or in products in which they are regularly applied. Furthermore, it was realized according to the invention that such darkening might be caused by the existence of peroxides.

It is known from the state of the art that the oxidation of aldehydes with oxygen initially forms peroxy acids which then oxidize further aldehyde to produce two moles of carboxylic acids per mole of intermediate peroxy acids. Such a mechanism is, for example, described in J.H. Teles et al., "Oxidation" in Ullmann's Encyclopedia of Industrial Chemistry, 2015, Wiley-VCH Verlag GmbH & Co. KGaA, DOI: 10.1002/14356007.a18_261.pub2, section 5.4.1 "Secondary reactions of radicals, peroxides, and other intermediates" in combination with section 2.2.8 "Carboxylic acids, saturated". It is therefore apparent that peroxy acids also occur as intermediates during the oxidation of the respective aldehydes with oxygen to the respective C₆₋₁₂ saturated aliphatic carboxylic acids. Peroxy acids are very reactive molecules. Even if only a small amount of them would remain after the oxidation in the crude carboxylic acid product and would not be properly separated off, the C₆₋₁₂ saturated aliphatic carboxylic acids might cause undesired properties such as a poor color stability.

CN 108047027 deals with the decomposition of such peroxides formed in the oxidation of 3,5,5-trimethylhexanal to 3,5,5-trimethylhexanoic acid (isononanoic acid). It was found that although its concentration is rather low, it is still high enough to cause problems in the rectification column since the peroxides may accumulate therein due to the difference in the boiling points of the peroxy acid and other components. Furthermore, the CN application describes that in the prior art, homogeneous catalysts in a relatively low concentration are used to decompose the peroxides. However, such homogeneous catalysts are very difficult to separate off and also cause the risks of slagging, clogging and explosions in the distillation column. To avoid such problems, the CN application teaches to heterogeneously catalyze the decomposition of the peroxides over a metal organic framework catalyst before entering the rectification column. It is taught to be crucial to only decompose the peroxides at a low temperature of 20 to 70°C since otherwise side reactions such as decarboxylation would appear and lower the yield and purity. Moreover, it is emphasized that the decomposition is very fast and therefore can be carried out at a high space velocity of 5 to 40 h⁻¹, which relates to a short residence time of 1.5 to 12 minutes.

However, it is generally disadvantageous to use a metal organic framework catalyst as peroxide decomposition catalyst. First of all, these metal organic framework catalysts are quite complex to produce. Second, the organic molecules that build the framework are susceptible to oxidation, especially in the presence of a peroxy acid and of the reactive radicals generated in their decomposition. In the presence of carboxylic acids, metal organic frameworks are known to lose active and/or framework metals as a result of bleeding. These leached metals would then cause the same problems in the rectification column as metals used as homogeneous catalyst. Furthermore, all these factors contribute to a short lifetime of the metal organic framework catalysts and thus to an additional increase of the complexity of the process by disposing the spent catalyst and providing fresh one.

In PCT application number PCT/EP2020/087,952 (based on the priority with the EP application number 20150845.4), it was recognized that the peroxy acids which remain after the oxidation of C₃₋₅ aldehydes in the C₃₋₅ saturated aliphatic carboxylic acid crude product can predominantly be decomposed by a thermal treatment before the distillative purification. The distilled C₃₋₅ saturated aliphatic carboxylic acids then only show a very low content of active oxygen, which in turn indicates a very low content of peroxides such as peroxy acids. The content of active oxygen is a quantitative measure of the amount of reactive oxygen which is capable to oxidize easily oxidable compounds such as salts of iodide(1-) to iodine(0) or iron(II) to iron(III).

It was recognized according to the invention that such a thermal treatment proposed in EP application number 20150845.4 for the production of C₃₋₅ saturated aliphatic carboxylic acids by oxidation of the corresponding C₃₋₅ aldehydes does not succeed in the production of C₆₋₁₂ saturated aliphatic carboxylic acids by oxidation of the corresponding C₆₋₁₂ aldehydes, at least not within a reasonable time of only a few hours. Although possible C₆₋₁₂ peroxy acids may be decomposed by such a thermal treatment and the distilled C₆₋₁₂ saturated aliphatic carboxylic acids show a low APHA color number, their content of active oxygen is still high and they tend to darken over time during storage, when subjected to thermal stress and in products in which they are regularly applied.

It was therefore an object of the present invention to find a process for the preparation of saturated aliphatic carboxylic acids with 6 to 12 carbon atoms by oxidation of the corresponding aldehydes with oxygen, which is able to produce the respective saturated aliphatic carboxylic acid in high yield and high purity, and particularly without or at least with a very low tendency to darken over time during storage, when the product is subjected to thermal stress and/or after its regular application, e.g. as additive in the manufacture of polymers. The process shall also be easy to operate, be safe in its performance and function stably over long operating times producing the saturated aliphatic carboxylic acids in a constant high quality.

We have surprisingly found a process for the preparation of a saturated aliphatic carboxylic acid with 6 to 12 carbon atoms by oxidation of the corresponding aldehyde with molecular oxygen, which comprises
(a) converting the corresponding aldehyde with molecular oxygen at a temperature of 0 to 120°C and an oxygen partial pressure of 0.02 to 2 MPa to obtain a liquid mixture containing the saturated aliphatic carboxylic acid, ≤ 2 mol-% of the corresponding aldehyde with respect to the saturated aliphatic carboxylic acid, and molecular oxygen;
(b) removing molecular oxygen from the liquid mixture obtained in step (a) to a content of ≤ 10 wt.-ppm based on the liquid mixture; and
(c) distilling the mixture obtained in step (b) in a distillation device containing a purification column, and withdrawing therefrom a purified distillate containing ≥ 95 wt.-% of the saturated aliphatic carboxylic acid based on the distillate.

The saturated aliphatic carboxylic acid with 6 to 12 carbon atoms, which is in the following shortly referred to as C₆₋₁₂ saturated aliphatic carboxylic acids, and for which the found production process is very suitable for, can be linear or branched as well as substituted or unsubstituted. Substituted C₆₋₁₂ saturated aliphatic carboxylic acids contain besides carbon and hydrogen one or more heteroatoms, for which halogen is mentioned as an example. Non-substituted C₆₋₁₂ saturated aliphatic carboxylic acids are preferred. Preferred examples broken down by their number of carbon atoms are

| | |
|---|---|
| for C₆: | hexanoic acid, 2-methylpentanoic acid, 3-methylpentanoic acid, 4-methylpentanoic acid, 2,3-dimethylbutanoic acid and 3,3-dimethylbutanoic acid; |
| for C₇: | heptanoic acid and 2-methylhexanoic acid; |
| for C₈: | octanoic acid, 2-methylheptanoic acid, 2-ethylhexanoic acid, 2-ethyl-4-methylpentanoic acid and 2-propylpentanoic acid; |
| for C₉: | nonanoic acid and 3,5,5-trimethylhexanoic acid; |
| for C₁₀: | decanoic acid, 2-propylheptanoic acid and 2-propyl-4-methylhexanoic acid; |
| for C₁₁: | undecanoic acid and 2-methyldecanoic acid; and |
| for C₁₂: | dodecanoic acid and 2-butyloctanoic acid. |

From the above-mentioned list, hexanoic acid, 2-methylpentanoic acid, heptanoic acid, octanoic acid, 2-ethylhexanoic acid, nonanoic acid, 3,5,5-trimethylhexanoic acid, decanoic acid, 2-propylheptanoic acid and dodecanoic acid are more preferred. Particularly preferred are C₈₋₁₂ saturated aliphatic carboxylic acids and within that octanoic acid, 2-ethylhexanoic acid, nonanoic acid, 3,5,5-trimethylhexanoic acid, decanoic acid, 2-propylheptanoic acid and dodecanoic acid. Very particularly preferred is 2-ethylhexanoic acid.

In the first step of the process according to the invention, which is named step (a), the corresponding aldehyde is oxidized with oxygen. Regarding the more preferred C₆₋₁₂ saturated aliphatic carboxylic acids as mentioned above, these aldehydes are n-hexanal for hexanoic acid, 2-methylpentanal for 2-methylpentanoic acid, n-heptanal for heptanoic acid, n-octanal for octanoic acid, 2-ethylhexanal for 2-ethylhexanoic acid, n-nonanal for nonanoic acid, 3,5,5-trimethylhexanal for 3,5,5-trimethylhexanoic acid, n-decanal for decanoic acid, 2-propylheptanal for 2-propylheptanoic acid and n-dodecanal for dodecanoic acid.

C₆₋₁₂ aldehydes can generally easily be prepared by diverse methods, depending on the accessibility of the primary product. One typical preparation method is the hydroformylation of the corresponding alkene having one carbon atom less than the desired aldehyde. As example, the hydroformylation of 1-pentene to n-hexanal is mentioned. Another typical preparation method is the aldol condensation to an alkenal as intermediate and its subsequent hydrogenation to the desired aldehyde. As example, the aldol condensation of n-butanal to 2-ethyl-2-hexenal and its subsequent hydrogenation to 2-ethylhexanal is mentioned. Last but not least, a third typical preparation method is the dehydrogenation of the corresponding alcohol. For this method, the dehydrogenation of 1-decanol to n-decanal is mentioned as an example.

The aldehyde which is intended to be oxidized can be used in diluted or pure form. If the aldehyde is used in a diluted form, the diluent shall preferably be a compound which is inert against an oxidation with oxygen, stable against the produced carboxylic acid and easily separated from the carboxylic acid by distillation. The afore mentioned necessity to separate the diluent from the carboxylic acid can be avoided if the corresponding carboxylic acid is used as diluent. However, since diluents raise the reaction volume and therefore lower the space time yield, and, if other diluents than the corresponding carboxylic acids are used, additionally may cause a contamination of the wanted carboxylic acid, it is preferred not to intentionally dilute the aldehydes. Preferably, the aldehyde is applied as a highly concentrated compound with an aldehyde content of preferably 80 to 100 wt.-%, more preferably of 80 to 100 wt.-%, particularly preferably of 95 to 100 wt.-% and very particularly preferably of 99 to 100 wt.-%.

Besides the preparation of only one specific saturated aliphatic carboxylic acid, the inventive process can also be applied for the preparation of mixtures of C₆₋₁₂ saturated aliphatic carboxylic acids. If such mixtures are prepared, especially mixtures of C₆₋₁₂ saturated aliphatic carboxylic acids with the same number of carbon atoms are then preferred. However, it is generally more preferred to prepare non-mixed C₆₋₁₂ saturated aliphatic carboxylic acids.

Particularly preferred is the preparation of 2-ethylhexanoic acid by oxidation of 2-ethylhexanal.

The oxidation of the aldehyde is performed with molecular oxygen. It can be used in pure form or diluted with other gases, for example in the form of air, an O₂/N₂ mixture or in form of mixtures with other inert gases.

The oxidation reaction may be carried out in the presence or absence of an oxidation catalyst and/or in the presence or absence of a selectivity improving additive. If oxidation catalysts are used, they will be homogeneous catalysts. As examples of homogeneous oxidation catalysts, salts of transition metals of groups 6 to 11 of the Periodic Table of the Elements are mentioned, preferably of the first row of these groups and most preferably Mn, Fe or Co salts. If selectivity improving additives are used, they will also be homogeneous. As examples of selectivity improving additives, salts of alkali metals, of alkaline earth metals and of transition metals of group 12 of the Periodic Table of the Elements are mentioned, preferably salts of Na, K, Mg, Ca, Zn or Cd, most preferably salts of K or Na and particularly preferably salts of K. The salts can be chosen from any salts soluble in the reaction mixture, but carboxylates, hydroxides, carbonates and hydrogen carbonates are preferred. The concentration of the homogeneous oxidation catalyst metals may vary in a broad range, but a metal content of 0.0001 to 0.1 wt.-% based on the reaction mixture is a typical content. The concentration of the homogeneous selectivity improving metals may also vary in a broad range, but a metal content of 0.01 to 5 wt.-%, preferably of ≥ 0.02 wt.-%, more preferably of ≥ 0.05 wt.-%, and preferably of ≤ 2 wt.-%, more preferably of ≤ 1 wt.-% and particularly preferably of ≤ 0.5 wt.-% based on the reaction mixture is a typical content. It is also possible to simultaneously use homogeneous catalyst metals and homogeneous selectivity improving metals.

Depending on the nature of the aldehyde, the presence of an oxidation catalyst and particularly the presence of selectivity improving metals will influence the range and type of the by-products. For example, alpha-branched aldehydes like 2-ethylhexanal tend to produce more formates as by-product if no selectivity improving metal is present. However, in the presence of a selectivity improving metal, particularly in the presence of a sodium or potassium salt and most particularly in the presence of a potassium salt, alpha-branched aldehydes produce much less unwanted formates. Thus, it is preferred for alpha-branched aldehydes to conduct the oxidation in the presence of a selectivity improving metal, preferably in the presence of a sodium or potassium salt and particularly preferably in the presence of a potassium salt. On the other hand, linear aldehydes like n-hexanal or n-decanal already form even in the absence of a selectivity improving metal only a very small amount of formates, so that the addition of a selectivity improving metal is not or only less relevant for the selectivity. Thus, it is preferred for linear aldehydes to conduct the oxidation in the absence of a selectivity improving metal.

As for homogeneous catalytic metals, they increase the reaction rate, but they have a detrimental effect on the selectivity. It is thus preferred to perform the oxidation in the absence of added homogeneous catalyst metals.

Irrespective of whether an oxidation catalyst is present or not, the oxidation reaction is performed at a temperature of 0 to 120°C and an oxygen partial pressure of 0.02 to 2 MPa. It is preferably carried out at a temperature of ≥ 10°C, more preferably at ≥ 20°C and particularly preferably at ≥ 30°C, and preferably at ≤ 100°C, more preferably at ≤ 80°C and particularly preferably at ≤ 60°C. With regard to the oxygen partial pressure, it is preferably carried out at an oxygen partial pressure of ≥ 0.05 MPa, more preferably of ≥ 0.1 MPa and particularly preferably of ≥ 0.11 MPa, and preferably of ≤ 1.5 MPa and more preferably of ≤ 1 MPa. The oxygen partial pressure can easily be determined by measuring the total pressure and multiplying by the concentration of O₂ in vol.-% determined by any suitable method known in the state of art.

Whereas the oxygen partial pressure may vary in a broad range based on the oxygen content of the oxygen source, the total pressure at step (a) is usually in the range of 0.01 to 5 MPa abs. The oxidation reaction is preferably carried out at a total pressure of ≥ 0.1 MPa abs and more preferably at a total pressure of ≥ 0.2 MPa abs. It is preferably carried out at a total pressure of ≤ 4 MPa abs and more preferably at a total pressure of ≤ 3 MPa abs.

At these conditions, the aldehydes are almost completely in the liquid phase and the oxidation reaction also takes place in the liquid phase.

The oxidation of the aldehyde in step (a) is typically performed in a reaction device, either batch-wise, semi-continuously or continuously. At a continuous operation, the aldehyde and oxygen are continuously fed to the reaction device and an adequate stream of the reaction mixture is continuously removed. The process conditions of the continuous operation, including the residence time, are selected in such a way that the desired conversion is achieved. At a batch operation, the reaction device is filled with aldehyde and oxygen added and, if required, replenished. After having achieved the desired conversion, the mixture is removed from the reaction device. A semi-continuous operation is characterized by adding aldehyde and oxygen together or intermittently to the reaction device over a specific period of time while the oxidation reaction already takes place. After a while, e.g. if the reaction device is more or less filled, the addition is stopped, and after having achieved the desired conversion, the mixture is removed from the reaction device.

The preferred operations for step (a) are batch operation and continuous operation, whereby the continuous operation is particularly preferred.

The reaction device, in which the oxidation of the aldehyde in step (a) is performed, may embrace one or more reaction apparatuses. In principle, suitable reaction apparatuses include devices which are suitable for carrying out exothermic, gas-liquid reactions and which can be operated discontinuously, semi-continuously or continuously. For a discontinuous process, stirred autoclaves or autoclaves with a jet loop type mixing are for example suitable. For a semi-continuous process, stirred vessels, trickle-bed reactors, and bubble column reactors are mentioned as possible examples. For a continuous process, stirred vessels, trickle-bed reactors, bubble column reactors, jet loop reactors and cascades of the above-mentioned reactors are mentioned as suitable examples. Preferred examples of suitable reactors are described in more detail in WO 2009/024,446 and WO 2009/024,549. If reactor cascades are used, as for example in a continuous process, 2 to 5, preferably 2 to 4, and particularly preferably 2 to 3 reactors connected in sequence.

It is preferred to use reaction apparatuses which enable an intensive gas-liquid mixing and a good distribution of the oxygen within the liquid reaction mixture.

Due to the formation of a considerable amount of reaction heat by the oxidation, it is necessary to remove the heat from the reaction zone. Depending on the concentration of the aldehyde and the oxygen fed into the reactor, it may suffice in a continuously performed process to remove the heat only with the reaction mixture and to control the temperature in the reactor by adding fresh aldehyde with a low temperature. However, for aldehydes in a higher concentration and an oxygen content in the order of air or above, it is normally required to cool the reaction liquid in the reactor. Such cooling may for example be performed by an externally cooled exterior wall of the reactor or by cooling tubes inside the reactor, through which a coolant is flowed or by using an external heat exchanger in an external loop.

According to the overall chemical equation 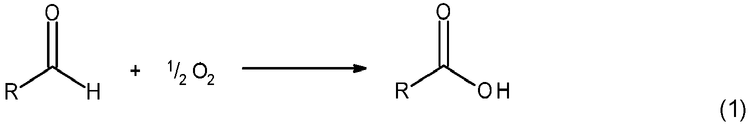 in which R denotes the C₅₋₁₁ radical of the C₅₋₁₁ aldehyde and the C₅₋₁₁ carboxylic acid, 0.5 mol of oxygen O₂ is stoichiometrically required to oxidize the aldehyde to the carboxylic acid. Although it would be possible to perform the oxidation with a deficit of oxygen with the result of a partial conversion and the existence of residual aldehyde in the reaction mixture, it is preferred to apply oxygen in stoichiometric or superstoichiometric amount. In order to ensure an adequate conversion at the one hand and to limit the gas load at the other hand, the oxidation reaction is preferably performed at a molar ratio of oxygen to aldehyde of 0.5 to 1. It is more preferably performed at a molar ratio of oxygen to aldehyde of ≥ 0.51 and particularly preferably of ≥ 0.52, and more preferably at ≤ 0.7, particularly preferably of ≤ 0.6 and very particularly preferably of ≤ 0.58.

It has been shown that for a continuous oxidation process, the use of a reactor cascade is particularly advantageous since it enables the stepwise addition of oxygen. The great advantages of such a stepwise addition of oxygen are a better control of the reaction heat and particularly a smaller gas fraction in the respective cascade stage. Therefore, it is particularly preferred to use such a reactor cascade of 2 to 3 reactors, whereby preferably the total amount of the aldehyde, and about 70 to 95% of the total oxygen are fed to the first reactor and the remaining 5 to 30% of the oxygen are either fed in total to the second reactor or further divided into two parts for the second and third reactor, whereby the fraction for the third reactor would then preferably be the smaller fraction.

Even though the oxygen is applied in stoichiometric or superstoichiometric amount, it would take a very long time to achieve an aldehyde conversion of nearly 100%. This would unnecessarily block the reaction equipment or make it extremely large. It is therefore advantageous to convert in step (a) the aldehyde until a residual aldehyde amount of ≤ 2 mol-% with respect to the saturated aliphatic carboxylic acid is achieved. Depending on the nature of the aldehyde, the concentration of the oxygen containing gas provided to the reaction device, and the process conditions, a residual aldehyde amount of ≤ 2 mol-% with respect to the saturated aliphatic carboxylic acid is generally achieved after a reaction time of 0.1 to 5 hours.

The composition of the reaction mixture of step (a) regarding the content of the saturated aliphatic carboxylic acid and the corresponding aldehyde can usually be determined by gas chromatography.

The mixture obtained in step (a) preferably contains ≤ 1.5 mol-%, more preferably ≤ 1 mol-%, particularly preferably ≤ 0.5 mol-% and very particularly preferably ≤ 0.3 mol-%, and preferably ≥ 0.05 mol-% and particularly preferably ≥ 0.1 mol-% of the corresponding aldehyde with respect to the saturated aliphatic carboxylic acid.

As the reaction time concerns, a time of ≥ 0.2 hours is preferred, ≥ 0.3 hours are more preferred and ≥ 0.5 hours are preferably preferred. Furthermore, a time of ≤ 8 hours is preferred, ≤ 4 hours are more preferred and ≤ 3 hours are particularly preferred.

After a mixture containing the saturated aliphatic carboxylic acid and ≤ 2 mol-% of the corresponding aldehyde with respect to the saturated aliphatic carboxylic acid as described above is obtained in step (a), the reaction liquid is then preferably separated from the remaining oxygen containing gas phase. In a batch process, this can, for example, be achieved by simply discharging the oxygen containing gas phase, and in a continuous process, for example, by simply removing the liquid reaction mixture from the reaction device.

Regarding possible by-products of the separated reaction liquid, it is known from the state of the art that peroxy acids are initially formed in the oxidation of aldehydes with oxygen, which then oxidize further aldehyde to produce two moles of carboxylic acids per mole of intermediate peroxy acids. The reaction steps are shown below 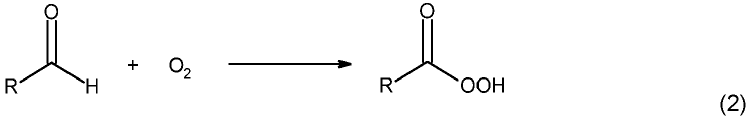 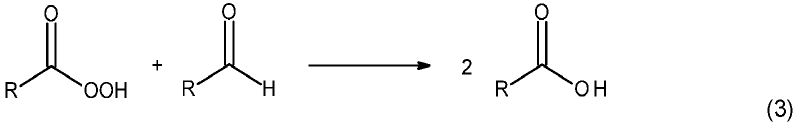 whereby R denotes a C₅₋₁₁ radical. Such acids are also expected to be formed in oxidation step (a) of the preparation of C₆₋₁₂ saturated aliphatic carboxylic acids, and, although the added aldehyde is normally not completely converted, expected to remain to some small extent in the liquid reaction mixture. Peroxy acids have a high oxidation potential and are able to cause a darkening of the carboxylic acids over time during storage, when the product is subjected to thermal stress and/or in products in which they are regularly applied, if not properly separated off. However, peroxy acids can, together with other components with a high oxidation potential, easily be characterized as so-called "active oxygen", which is a quantitative measure of the amount of reactive oxygen. The term "active oxygen" is already known and used in the state of the art, and is for example described in A. Uhl et al., "Peroxy Compounds, Organic" in Ullmann's Encyclopedia of Industrial Chemistry, 2017, Wiley-VCH Verlag GmbH & Co. KGaA, DOI: 10.1002/14356007.a19_199.pub2, chapter 10 "Analytical Determination". The amount of active oxygen of a sample is generally determined by adding a defined amount of an easily oxidable compound, such as salts of iodide(1-) or iron(II), to a defined amount of the sample. The present reactive oxygen oxidizes the oxidable compound and the amount of the oxidized oxidable compound afterwards determined by titration.

Since the boiling points of the peroxy acids do generally not largely deviate from those of the corresponding acids and therefore generally cannot easily be separated off from the corresponding acids, and based on the knowledge that peroxy acids as impurities in the distilled acids would cause a darkening over time during storage, when subjected to thermal stress and in products in which the acids are regularly applied, it was initially assumed that such peroxy acids are also responsible for the darkening of C₆₋₁₂ saturated aliphatic carboxylic acids. Based on the teaching in EP application number 20150845.4 relating to the preparation of C₃₋₅ saturated aliphatic carboxylic acids, which was already mentioned in the introductory summary of the state of the art, peroxy acids are expected to easily decompose by a thermal treatment, which is taught to be performed before the distillative purification. However, it was recognized according to the invention that such a thermal treatment does not succeed in the preparation of C₆₋₁₂ saturated aliphatic carboxylic acids, at least not within a reasonable time of only a few hours. First, the active oxygen content in the C₆₋₁₂ saturated aliphatic carboxylic acid crude products which have been prepared according to step (a) does only slightly decrease by such a thermal treatment. Second, although possible C₆₋₁₂ peroxy acids may decompose by such a thermal treatment and the distilled C₆₋₁₂ saturated aliphatic carboxylic acids show a low APHA color number, their content of active oxygen is still high and they tend to darken over time during storage, when subjected to thermal stress and in products in which they are regularly applied.

It was surprisingly found that in a subsequent distillation of the thermally treated product as well as in a distillation of the non-thermally treated product the large majority of the components, which cause the active oxygen content, are enriched in the bottom fraction, and only a small but still noteworthy portion was found in the distilled C₆₋₁₂ saturated aliphatic carboxylic acid. Both findings led to the important conclusion that the active oxygen content in the C₆₋₁₂ saturated aliphatic carboxylic acid crude products is mainly caused by other components than peroxy acids. A deeper investigation on the nature of these other components revealed that these other components are mainly alkyl hydroperoxides with the general formula (4) 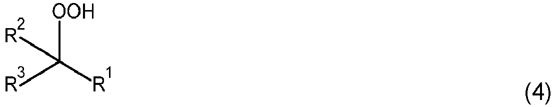 in which R¹ denotes a C₁₋₁₁ radical with a COOH group, R² denotes a C₁₋₁₀ radical or H, and R³ denotes a C₁₋₅ radical or H, whereas the sum of the carbon atoms of R¹, R² and R³ is 5 to 11, depending on the applied aldehyde. The hydroperoxy group may be located at any carbon atom of the C₆₋₁₂ saturated aliphatic carboxylic acid irrespective of whether the carbon atom is a primary, secondary or tertiary carbon atom, but except for the COOH group. However, tertiary carbon atoms as well as the carbon atom in alpha position to the COOH group are particularly prone for peroxidation.

Alkyl hydroperoxides are able to cause a darkening of the carboxylic acids over time during storage, when subjected to thermal stress and/or in products in which they are regularly applied, if not properly separated off.

Alkyl hydroperoxides of saturated aliphatic carboxylic acids are known to have a significantly higher boiling point than the respective saturated aliphatic carboxylic acid. The following list shows the boiling points of some 2-hydroperoxy acids compared with those of the respective acids. Their boiling points at atmospheric pressure have been estimated by SciFinder, which is an electronic database of chemical and bibliographical information provided by the American Chemical Society, to be

| | |
|---|---|
| 195 ± 8°C | for 2-methylpentanoic acid; |
| 295 ± 23°C | for 2-methyl-2-hydroperoxypentanoic acid; |
| 239 ± 3°C | for octanoic acid; |
| 327 ± 25°C | for 2-hydroperoxyoctanoic acid; |
| 306 ± 10°C | for 2-ethyldecanoic acid; and |
| 373 ± 25°C | for 2-ethyl-2-hydroperoxydecanoic acid. |

Such significant differences in the boiling points should facilitate an easy separation by distillation. The high content of active oxygen in the bottom fraction found by the above-mentioned investigation is also a strong indication for that. However, based on that, it is all the more astonishing that also a small but noteworthy portion of the active oxygen could still be found in the distilled C₆₋₁₂ saturated aliphatic carboxylic acid. A slippage of the respective hydroperoxyl acids during the distillation is very unlikely due to their high boiling points.

To be more precise regarding the information value of the active oxygen content, its measuring method is explained in more detail. According to the present invention, the determination of active oxygen is preferably performed by oxidation of iodide(1-). This analytical method is called iodometry and is well known by the person skilled in the art. However, it is also roughly explained below.

At the iodometry, a defined amount of an aqueous solution of potassium iodide in acetic acid is added to a defined amount of the sample at room temperature and stirred in order to oxidize the iodide(1-) to elemental iodine. The amount of added potassium iodide relates to the expected amount of active oxygen and can be estimated by preliminary measures. For the iodometric measurement, the amount of the added iodide(1-) has to be a bit higher than the amount oxidized to elemental iodine. The elemental iodine is then titrated with sodium thiosulfate in order to determine the amount of elemental iodine which was formed by the previous oxidation. As an indicator, starch is typically used, which is violet as long as elemental iodine is present and which gets colorless when all the elemental iodine was reduced to iodide. Alternatively, also a platinum electrode can be used. Based on the amount of added potassium iodide and the amount of elemental iodine formed by the oxidation, the amount of the oxidized iodide(1-) can be calculated. According to the formal equation

2I⁻+2H⁺+"O"→I₂+H₂O (5)

and the more detailed equations for peroxy acids and alkyl hydroperoxides 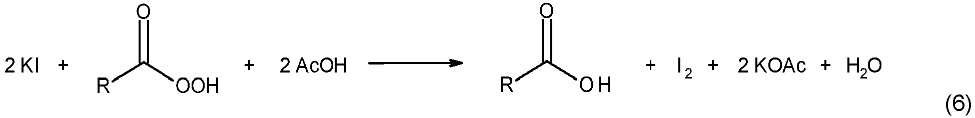 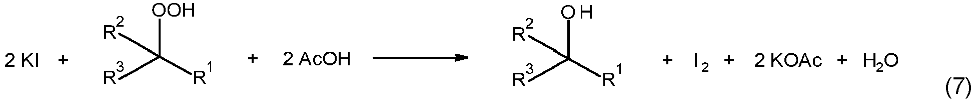 two moles of iodide(1-) react in the presence of acetic acid with one mole of active oxygen atoms. Active oxygen is indicated in formula (5) by "O" and is part of the peroxo group in formulas (6) and (7). It is reduced to water. In equations (6) and (7), "Ac" stands for an acetyl group and the radicals R, R¹, R² and R³ have the meanings as described for formulas (2)/(3) and (4), respectively. The active oxygen content of the sample is the weight fraction of the active oxygen atoms based on the weight of the sample and specified in wt.-% or wt.-ppm.

The content of active oxygen can easily be converted into an equivalent content of the peroxide compound, assuming that the active oxygen would solely be bound in the C₆₋₁₂ saturated aliphatic carboxylic acid as hydroperoxide. The conversion can be made by multiplying the determined active oxygen content by the ratio of the molar mass of the hydroperoxy acid to the molar mass of an oxygen atom. In case of 2-ethylhexanoic acid, for example, the multiplication factor is 176.2/16.0 = 11.0125.

For the sake of completeness, it is also mentioned that the amount of active oxygen in carboxylic acid containing samples can basically also be determined by physical methods such as ¹³C-NMR.

However, within the present invention, active oxygen is understood as the mass of oxygen present in the sample which is capable to oxidize iodide(1-) in aqueous acetic acid medium at room temperature and atmospheric pressure to elemental iodine.

The active oxygen content of the mixture obtained in step (a) is typically 0.02 to 1 wt.-% based on the mixture, preferably ≥ 0.03 wt.-% and more preferably ≥ 0.05 wt.-%, and preferably ≤ 0.8 wt.-% and more preferably ≤ 0.5 wt.-%.

After intensive studies of the astonishing behavior of the C₆₋₁₂ saturated aliphatic carboxylic acid crude product mixture, it was surprisingly found that the existence of molecular oxygen in the crude product mixture mainly causes the noteworthy content of active oxygen in the distilled C₆₋₁₂ saturated aliphatic carboxylic acid.

In the oxidation step (a), a part of the molecular oxygen, depending on its partial pressure, the temperature of the liquid mixture and the chemical composition of the liquid mixture, remains unreacted in the liquid mixture as physically dissolved molecular oxygen. Its concentration in the liquid mixture obtained by step (a) is generally > 10 wt.-ppm to ≤ 1 wt.-% based on the liquid mixture. A content of ≤ 10 wt.-ppm based on the liquid mixture is generally not achieved since a small amount of molecular oxygen remain unreacted in the liquid mixture, and a content of > 1 wt.-% is not achieved in practice due to its limited solubility. As the mentioned concentration of molecular oxygen relates to the liquid mixture obtained in step (a), the value refers to the reaction conditions at the end of the conversion just before the liquid mixture leaves the reaction device. The measurement of the concentration of molecular oxygen in the liquid mixture is therefore preferably performed under the above-mentioned conditions just before the liquid mixture leaves the reaction device. However, it can alternatively also be measured at a lower pressure, e.g. at atmospheric pressure, and the measured value corrected under the consideration of recognized laws such as Henry's law for the pressure dependence. The concentration of molecular oxygen in the liquid mixture obtained in step (a) is preferably ≥ 20 wt.-ppm and more preferably ≥ 50 wt.-ppm. Regarding its upper value, it is generally ≤ 1 wt.-% (or ≤ 10000 wt.-ppm expressed in ppm scale), preferably ≤ 5000 wt.-ppm and more preferably ≤ 2500 wt.-ppm, particularly preferably ≤ 1000 wt.-ppm and very particularly preferably ≤ 750 wt.-ppm.

The concentration of molecular oxygen in the liquid mixture obtained in step (a) can easily be determined. Useful devices are, for example, optical sensors which are often used in water analysis. As a possible example, an optical fluorescence sensor is mentioned. Optical fluorescence sensors are state of the art and the person skilled in the art knows how to calibrate and to use them. Such sensors are very specific to oxygen and very sensitive to oxygen. They can even measure very low concentrations down to 0.01 wt.-ppm. Since such optical fluorescence sensors are to some extent heat and pressure resistant, they can also be used for in-line measurements.

Having explained that the liquid C₆₋₁₂ saturated aliphatic carboxylic acid crude product mixtures obtained by step (a) contain hydroperoxides and at the most a very small amount of the respective as by-products as well as unconverted, dissolved molecular oxygen, it is important to mention that the term active oxygen does not embrace molecular oxygen. This is due to the fact that molecular oxygen reacts only very slowly under the conditions used for the determination of active oxygen in samples containing hydroperoxides and furthermore it is good practice to perform the determination under inert gas to minimize any interference from molecular oxygen. As advantageous consequence of that, active oxygen and molecular oxygen can be separately determined and evaluated.

Based on all the above-mentioned surprising findings, it was then found that a darkening of the carboxylic acids over time during storage, when subjected to thermal stress and/or in products in which they are regularly applied, can be avoided or at least significantly reduced, if the molecular oxygen present in the liquid C₆₋₁₂ saturated aliphatic carboxylic acid crude product mixture obtained by step (a) is removed in a subsequent step, which is named step (b), to a content of ≤ 10 wt.-ppm based on the liquid mixture, before it is distilled to isolate the C₆₋₁₂ saturated aliphatic carboxylic acid. For the sake of completeness, it is mentioned that there can be further steps between step (a) and (b), including, for example, decreasing or increasing temperature or pressure, or removing low boilers by distillation.

There are several possibilities for removing molecular oxygen in the liquid crude product mixture to a content of ≤ 10 wt.-ppm. One possibility is to give the mixture a long time to allow the dissolved molecular oxygen to react with the aldehyde still present. This can, for example, be performed in a pipe through which the mixture flows and which enables the intended residence time, or in one or more residence time vessels which are connected in series. Although such a procedure does neither require the addition of further compounds nor the adjustment of specific conditions such as an increased temperature, it is not the preferred way due its long time requirement of typically much more than 10 hours and rather more than 50 hours or even more than 100 hours.

As preferred possibility for removing molecular oxygen to a content of ≤ 10 wt.-ppm, the stripping of the liquid crude product mixture obtained in step (a) with an inert gas is mentioned.

Basically, the stripping can be performed in a container, also called stripper, in which the liquid crude product mixture can be contacted with the inert gas in order to transfer the dissolved molecular oxygen to the gas phase of the inert gas and to remove it together with the gas phase. For a continuous preparation process, the liquid crude product mixture obtained in step (a) is continuously fed to a stripper, in which the inert gas can be passed through the mixture. Principally, such strippers can be of different shapes so that principally any kind of containers known in the state of the art for contacting a gas with a liquid phase can be used, but preferably so-called stripping towers are preferred. Stripping towers are characterized by a ratio of their length to their mean diameter of > 1, preferably ≥ 5, more preferably ≥ 8, and preferably ≤ 15. Whereas the inert gas is fed to the lower zone of the stripping tower to bubble to the upper zone, the liquid crude product mixture can either be fed to the lower zone causing a co-current flow or the upper zone causing a counter-current flow. Preferred is the continuous preparation of the C₆₋₁₂ saturated aliphatic carboxylic acid with the stripping in step (b) in a counter-current flow. Irrespective of whether the stripping is performed in co-current or counter-current flow, it is favorable to improve the contact between the gas and liquid phase. This can easily be attained by random or structured packings or trays within the stripper. Such internals for promoting the gas/liquid contact are well known by the person skilled in the art and can easily be selected.

Alternatively, the molecular oxygen can also be removed by spraying the liquid crude product mixture into the top of an apparatus with free space such as a vertical tower, in which inert gas is fed from the bottom in a counter-current flow.

For a discontinuous or semi-continuous preparation process, the liquid crude product mixture, for example, can be kept in the reactor in which the conversion of the aldehyde in step (a) took place and the inert gas passed through the obtained liquid crude product mixture, preferably after the gas phase of the molecular oxygen containing oxidation gas was discharged. It is also possible to transfer the liquid crude product mixture from the reactor of step (a) to another container, also called stripper, in which the inert gas is passed through the mixture. For such strippers, the above description relating to the continuous preparation process applies in an analogous manner, but with the exception that the liquid crude product mixture is normally a stationary phase.

Suitable inert gases for the stripping are substances which are gaseous under the conditions under which the stripping is performed, which do not react with the liquid crude product mixture and which are essentially free of molecular oxygen. Although the tolerable concentration of molecular oxygen in the inert gas may depend on the nature of the C₆₋₁₂ saturated aliphatic carboxylic acid and the conditions under which the liquid crude product mixture is stripped, the inert gas preferably has a content of molecular oxygen of ≤ 5 vol.-ppm, more preferably of ≤ 2 vol.-ppm and particularly preferably of ≤ 1 vol.-ppm. Small amounts of molecular oxygen may, for example, be present in the inert gas as impurities from their preparation or purification, respectively. As possible inert gases, nitrogen, hydrogen, carbon dioxide, carbon monoxide, nitrous oxide and noble gases such as helium, neon, argon and krypton are mentioned. The inert gas can be provided in pure form as well as a mixture of two or more different inert gases. Due to its availability, nitrogen is preferred.

The stripping in step (b) can be performed at a broad temperature and pressure range. It is preferably performed at a temperature of 0 to 150°C and a pressure of 0.0001 to 10 MPa abs. More preferably, it is performed at a temperature within the range of the temperature at which in step (a) the liquid crude product mixture leaves the reactor device and the temperature at which the stripped mixture is fed to the distillation device in step (c). The stripping is particularly preferably performed at ≥ 25°C, and particularly preferably at ≤ 100°C and very particularly preferably at ≤ 60°C, which is particularly advantageous for the liquid C₆₋₁₂ saturated aliphatic carboxylic acid crude product mixture in terms of the avoidance or at least minimization of undesired reactions between the molecular oxygen and the liquid mixture and particularly the carboxylic acid already formed. With regard to the pressure, it is more preferably performed at a pressure within the range of the pressure at which in step (a) the liquid crude product mixture leaves the reactor device and the pressure at which the stripped mixture is fed to the distillation device in step (c). The above-mentioned broad pressure range already indicates the great flexibility in the selection of the pressure. Depending on the available facilities and infrastructure at the plant site, the advantageous of a stripping under vacuum conditions or at atmospheric pressure and above may prevail. In case of sufficient vacuum power, stripping under vacuum conditions at 0.0001 to < 0.1 MPa abs is generally preferred since the required amount of stripping gas is low and the stripping highly efficient. Alternatively, a stripping at ≥ 0.1 MPa abs has the advantage that no vacuum power is required. However, stripping at atmospheric pressure or above is preferably performed at ≤ 0.2 MPa abs, since the volume of the inert gas is still high enough to particularly advantageously bubble through the liquid mixture.

The amount of the inert gas to be applied for the removal of a specific amount of molecular oxygen by stripping can vary in a wide range. It was surprisingly found that the stripping is highly effective and that a low molar amount of inert gas per molar amount of molecular oxygen to be removed already suffices. Therefore, the stripping in step (b) is preferably be performed at a molar ratio between the inert gas fed to the stripper and the molecular oxygen to be removed of ≥ 0.25 mol/mol. However, the molar ratio between the inert gas fed to the stripper and the molecular oxygen to be removed is preferably ≤ 1 mol/mol and more preferably ≤ 4 mol/mol. Regarding the amount of inert gas per unit of time, it shall be low enough to not exceed the flooding point of the stripper. Based on the geometry of the stripper, the amount of the liquid crude product mixture fed to the stripper in case of a continuous operation, the process conditions such as temperature and pressure, and the physical properties of the mixture, the person skilled in the art can calculate or alternatively experimentally determine the maximum tolerable amount of inert gas per unit of time for the respective case.

Based on the above-mentioned low molar ratio between the inert gas fed to the stripper and the molecular oxygen to be removed, the stripping in a continuously operated stripping tower can normally be performed within 1 to 10 minutes, preferably ≥ 2 minutes and preferably ≤ 5 minutes. Stripping in a batch operation, in which the liquid crude product mixture is already placed in the apparatus in which it is then stripped with inert gas, usually requires more time. For example, stripping in a batch operated bubble column would normally require some minutes to even some hours to attain the desired removal of molecular oxygen.

By the inventive removal of molecular oxygen from the liquid mixture obtained in step (a), the content of molecular oxygen is reduced in step (b) to ≤ 10 wt.-ppm, preferably to ≤ 5 wt.-ppm, more preferably to ≤ 2 wt.-ppm, particularly preferably to ≤ 1 wt.-ppm and very particularly preferably to ≤ 0.5 wt.-ppm. The content of molecular oxygen can even be reduced to below its detection limit, but a small content of ≥ 0.05 wt.-ppm typically remains. The concentration of molecular oxygen in the liquid mixture obtained in step (b) can easily be determined in the same way as already described for step (a).

The liquid, molecular oxygen depleted C₆₋₁₂ saturated aliphatic carboxylic acid crude product mixture obtained in step (b) is then transferred to step (c). For the sake of completeness, it is mentioned that the transfer may include further steps between step (b) and (c), as for instance the decreasing or increasing of temperature or pressure.

In step (c), the mixture obtained in step (b) is distilled in a distillation device containing a purification column, and withdrawing therefrom a purified distillate containing ≥ 95 wt.-% of the saturated aliphatic carboxylic acid. The term purification column refers to a distillation column in which the purified distillate of the C₆₋₁₂ saturated aliphatic carboxylic acid is obtained. In addition to the purification column, the distillation device can also contain further distillation columns, as for instance distillation columns for the removal of light boiling components or columns for the work-up of high boilers. The total number of distillation columns is not limited, but generally 1 to 5, preferably 1 to 4, more preferably 1 to 3 and particularly preferably 1 to 2 distillation columns are used. Beside the distillation columns as such and their internals, the term distillation device also embraces their periphery such as the piping, heat exchangers, reboilers, condensers, reflux drums and the like.

The distillative separation of the C₆₋₁₂ saturated aliphatic carboxylic acid can generally be performed continuously or discontinuously.

In case of a discontinuous or semi-continuous oxidation process in step (a), it is usually advantageous to also discontinuously perform step (b) and (c). For such a discontinuous distillation, the distillation device preferably contains only one distillation column which coincidentally constitutes the purification column. Therein, the low boilers are at first separated off overhead and the C₆₋₁₂ saturated aliphatic carboxylic acid subsequently obtained as a further fraction. It may also be favorable to perform the removal of molecular oxygen and the subsequent distillation in one and the same distillation device, in order to minimize the number of the required apparatuses and to simplify the process.

In case of a continuous oxidation process in step (a), a continuous as well as a discontinuous removal of molecular oxygen in step (b) and distillation in step (c) may be advantageous and both are basically equally preferred. However, the advantages of a continuous distillation generally prevail for larger capacities and the advantages of a discontinuous distillation for smaller capacities. Normally, a continuous distillation is preferred for plants with a capacity of > 1000 tons of a C₆₋₁₂ saturated aliphatic carboxylic acid per year, whereas a discontinuous distillation is preferred for plants with a respective capacity of ≤ 1000 tons per year. Whereas for a discontinuous distillation, the distillation device preferably contains only a single distillation column, a continuous distillation is generally be performed either in a single distillation column or in an interconnection of multiple distillation columns. Regarding a discontinuous distillation, the explanations of the preceding paragraph apply accordingly. Regarding a continuous distillation, this can, for example, be done in one distillation column which then coincidentally constitutes the purification column and in which the low boilers are separated off overhead and the C₆₋₁₂ saturated aliphatic carboxylic acid withdrawn as a side stream. The high boilers are withdrawn as bottom stream. The continuous distillation can also be performed in two or more interconnected distillation columns. If two interconnected distillation columns are used, the low boilers are usually separated off in the first distillation column and the bottoms containing the C₆₋₁₂ saturated aliphatic carboxylic acid and the high boilers transferred into the second distillation column, which constitutes the purification column and in which the C₆₋₁₂ saturated aliphatic carboxylic acid is withdrawn overhead, leaving the high boilers as bottom product. In a further and unfavored variant with two interconnected distillation columns, the saturated aliphatic carboxylic acid together with the low boilers are withdrawn in the first distillation column overhead and thus already separated off from the high boilers in the first distillation column, but have then to be purified in the second distillation column, which would require a further energy-intensive evaporation of the saturated aliphatic carboxylic acid. Instead of using two interconnected distillation columns, also a divided wall column or an equivalent Petlyuk configuration can be used. For the sake of completeness, the use of three or more distillation columns is also mentioned, for example in order to also separate the light boilers into different fractions. The use of three or more distillation columns may also be of interest, if two or more different C₆₋₁₂ saturated aliphatic carboxylic acids are prepared together since this normally requires one separate purification column for each C₆₋₁₂ saturated aliphatic carboxylic acid.

Irrespective of whether only one distillation column or an interconnection of two or more distillation columns is used, they can be, and are preferably equipped with separation efficiency facilitating internals, such as structured packings, random packings or trays. The number of separation stages required is mainly dependent on the separation task, and particularly on the differences of the boiling points of the saturated aliphatic carboxylic acid in relation to those of the low boilers and the high boilers, as well as on the aimed purity of the saturated aliphatic carboxylic acid.

With respect to the operation conditions in step (c), it is recommended to not expose the C₆₋₁₂ saturated aliphatic carboxylic acid to a temperature exceeding 170°C since it was recognized according to the invention that a higher temperature increasingly facilitates the formation of unwanted by-products such as the formation of anhydrides. Therefore, the temperatures in the distillation columns in which the C₆₋₁₂ saturated aliphatic carboxylic acids are processed are preferably ≤ 170°C, and particularly the temperature in the purification column is preferably ≤ 170°C, more preferably ≤ 150°C and particularly preferably ≤ 130°C. This can be easily achieved by selecting an appropriate pressure. Since even the lowest boiling C₆₋₁₂ saturated aliphatic carboxylic acid has a boiling point at atmospheric pressure of above 190°C, the distillation in step (c) is preferably performed at 0.1 to 99 kPa abs, which relates to all distillation columns in which the C₆₋₁₂ saturated aliphatic carboxylic acid is processed. The distillation is more preferably performed at a pressure of ≥ 0.5 kPa abs, particularly preferably at ≥ 1 kPa abs, and more preferably at ≤ 50 kPa abs and particularly preferably at ≤ 20 kPa abs. If more than one distillation column is used, each distillation column can be operated at a different pressure. Considering the above-mentioned pressure ranges, the distillation in step (c) is generally carried out at a temperature of ≥ 0°C, preferably at ≥ 25°C and more preferably at ≥ 40°C.

Since very low pressures of ≤ 1 kPa abs lead to low distillation temperatures of generally ≤ 100°C, and such low distillation temperatures significantly reduce the detrimental effect of molecular oxygen, the distillation at very low pressures may be a specific option to obtain a particularly pure C₆₋₁₂ saturated aliphatic carboxylic acid or alternative may be an option to tolerate a slightly higher content of molecular oxygen. However, such a very low pressure variant is not a preferred option since at these very low pressures the distillation rate declines rapidly causing either a very low throughput or requiring an unfavorable large diameter of the tower.

Based on the above-mentioned findings and relationships, the purification column is preferably be operated at a pressure of 0.1 to 99 kPa abs and a temperature of 0 to 170°C.

As a logical consequence of the surprising finding that the darkening of the carboxylic acids over time during storage, when subjected to thermal stress and/or in products in which they are regularly applied, can be avoided or at least significantly reduced, if the molecular oxygen present in the liquid C₆₋₁₂ saturated aliphatic carboxylic acid crude product mixture obtained by step (a) is removed in step (b) to a content of ≤ 10 wt.-% based on the liquid mixture before it is distilled to isolate the C₆₋₁₂ saturated aliphatic carboxylic acid, it is self-evident that it would be counterproductive to infiltrate molecular oxygen after its removal in step (b), including the transfer to step (c), during the distillation in step (c) and thereafter. The infiltration of molecular oxygen can be avoided or at least reduced to a very low extent if the apparatuses in which the C₆₋₁₂ saturated aliphatic carboxylic acid is processed are tight or at least largely tight under the operation conditions. This is particularly advisable for sections working under vacuum, especially for the distillation columns and particularly especially for the purification column. A high tightness of the used columns, particularly of vacuum distillation columns but also of the stripper in case it is operated at reduced pressure, can be achieved by different technical means known to the person skilled in the art. Such means are, for example, the use of high-quality seals with low leakage rates or even welded joints, the use of inert gas flushed flange protectors, or the minimization of the number of the flanges. As a general rule, the more of such means are implemented, the lower the leakage rate of the distillation column will be.

Although it is desirable to avoid any infiltration of molecular oxygen in the step (c), a low amount of infiltrated molecular oxygen in relation to the amount of the molecular oxygen depleted C₆₋₁₂ saturated aliphatic carboxylic acid crude product mixture processed in step (c) does not significantly impair the purity of the C₆₋₁₂ saturated aliphatic carboxylic acid distillate and generally still allows to obtain a low APHA color number after the tempering procedure specified in step (c) within the specified range. As consequence, large distillation columns of technical size, which are for instance designed for the distillation of 1000 kg per hour or more, can generally tolerate a greater absolute amount of infiltrated molecular oxygen per unit of time than small distillation columns of laboratory size, which for instance handle only a small amount of only 1 kg per hour or less. Moreover, the ratios between the surfaces of the sealings and the inner volume of the distillation columns are much lower for large columns of industrial size than for small scale laboratory columns. It was recognized according to the invention that an amount of molecular oxygen infiltrated in the distillation device of step (c) in the range of the amount of molecular oxygen fed to the distillation device by the molecular oxygen depleted C₆₋₁₂ saturated aliphatic carboxylic acid crude product mixture of step (b) can be tolerated.

Although all parts of the distillation device of step (c) in which the C₆₋₁₂ saturated aliphatic carboxylic acid is processed shall advantageously be as tight as reasonable possible, specifically the tightness of the purification column is more sensitive on the purity of the C₆₋₁₂ saturated aliphatic carboxylic acid distillate. This does particularly apply for purification columns operated under vacuum conditions. The tightness, or in other words the leakage rate, of a distillation column can be determined by measuring the amount of molecular oxygen infiltrated during the distillation and by comparing these values with the amount of molecular oxygen fed to the distillation column via the feed stream. For vacuum columns, this can be done by measuring the amount of the off-gas of the vacuum unit and the concentration of molecular oxygen therein.

It is preferred that the purification column which is operated at a pressure of 0.1 to 99 kPa abs and a temperature of 0 to 170°C is at least so tight that the amount of molecular oxygen withdrawn by the off-gas of its vacuum unit is ≤ 2 times the amount of molecular oxygen fed to the distillation device by the mixture obtained in step (b). More preferably, the amount of molecular oxygen withdrawn by the off-gas of its vacuum unit is ≤ 1.8 and particularly preferably ≤ 1.5, and regarding the lower limit ≥ 1 times the amount of molecular oxygen fed to the distillation device by the mixture obtained in step (b). Regarding a discontinuous distillation, the term amount stands for the absolute amount during the discontinuous distillation, whereas for a continuous distillation, the term amount relates to the amount per unit of time. The amount of molecular oxygen of the molecular oxygen depleted C₆₋₁₂ saturated aliphatic carboxylic acid crude product mixture fed to the distillation device can easily be determined by measuring the concentration of molecular oxygen as already described above, for instance by using an optical molecular oxygen sensitive sensor, and considering the total amount of the product mixture in case of a discontinuous distillation, or in relating it to the amount of the product mixture fed to the purification column per unit of time in case of a continuous distillation. The amount of molecular oxygen withdrawn by the off-gas of the vacuum unit of the purification column can easily be determined by measuring the concentration of molecular oxygen of the off-gas of the vacuum unit, preferably at the pressure side, and by determining the amount of the off-gas, for example by using a flow meter. For a discontinuous distillation, the total amount, and for a continuous distillation, the relative amount per unit of time is calculated.

Since the above-mentioned method for determining the tightness or leakage rate of vacuum columns requires, beside the concentration of molecular oxygen in the column off-gas, also the amount of the off-gas, it may sometimes be difficult to get a reliable value for the amount of the off-gas since its amount is sometimes very low and therefore difficult to measure. Therefore, an alternative determination method was developed in which the amount of the column off-gas is not required. This alternative method takes into consideration that not absolutely tight vacuum columns suck some surrounding air into the column. Since air contains argon in a fixed ratio to molecular oxygen, the concentration of argon in the column off-gas can be used as an indirect measure for the concentration of molecular oxygen which is based on the infiltration of air. The concentration of molecular oxygen in air is 20.95 vol.-% and the concentration of argon is 0.93 vol.-% which corresponds to a molar ratio of Ar/O₂ of 0.0444. An absolutely tight column which is fed with an argon-free and molecular oxygen depleted C₆₋₁₂ saturated aliphatic carboxylic acid crude product mixture leads to an Ar/O₂ molar ratio of 0 (zero) since no argon is present in the column off-gas. Conversely, a leaky column which is fed with a product mixture free of argon and molecular oxygen leads to an Ar/O₂ molar ratio of 0.0444 which is the theoretical value for air. Depending on the amount of molecular oxygen fed with a product mixture to the distillation column, the Ar/O₂ molar ratio for a leaky column will be lower than the value for pure air. If the amount of infiltrated molecular oxygen would be the same as the amount of molecular oxygen fed to the distillation column with the molecular oxygen depleted product mixture, the Ar/O₂ molar ratio would be 0.0222. The tighter the column, the lower the Ar/O₂ molar ratio and vice versa, whereas a concentration of molecular oxygen in the column off-gas of 0 vol.-% relates per definition to an absolutely tight distillation column.

According to this alternative determination method, it is preferred that the purification column which is operated at a pressure of 0.1 to 99 kPa abs and a temperature of 0 to 170°C is at least so tight that the molar ratio n(Ar off-gas) / n(O₂ off-gas) in the off-gas of the vacuum unit of the purification column is 0 to 0.0222, wherein n(Ar off-gas) is the molecular amount of argon in the off-gas, corrected by the molecular amount of argon possibly fed to step (c) by the mixture obtained in step (b), and n(O₂ off-gas) is the molecular amount of molecular oxygen in the off-gas. More preferably, the molar ratio n(Ar off-gas) / n(O₂ off-gas) is ≤ 0.0197 and particularly preferably ≤ 0.0148. For a discontinuous distillation, the term amount stands for the absolute amount during the discontinuous distillation, whereas for a continuous distillation, the term amount relates to the amount per unit of time. Regarding the measurement of the molecular oxygen in the off-gas of the vacuum unit, the above explained determination methods can be applied. The molecular amount of argon in the off-gas of the vacuum unit can, for example, easily be determined by a gas chromatographic analysis of the off-gas at the pressure side. For instance, portable gas chromatographs may be used. If the mixture obtained in step (b) would already contain argon, for whatever reason, its amount could also be determined gas chromatographically and used for a correction of the n(Ar off-gas) value.

Furthermore, it was realized according to the invention that the position of the feed point at which the feed is added to the purification column in relation to the position of the sampling point at which the C₆₋₁₂ saturated aliphatic carboxylic acid is withdrawn also affects the temper stability of the C₆₋₁₂ saturated aliphatic carboxylic acid, particularly if the distillation column is not a divided wall column. If the position of the feed point of a distillation column which is not a divided wall column is above the position of the sampling point, the C₆₋₁₂ saturated aliphatic carboxylic acids are generally less temper stable than vice versa. Therefore, it is preferred that in step (c) the feed to the purification column, which is not a divided wall column, is added at a lower point than the purified distillate is withdrawn. Taking the total external height of the purification column as 100%, the distance between the horizontal levels of the feed point and the sampling point which is at a higher level than feed point is preferably ≥ 1%, more preferably ≥ 2%, particularly preferably ≥ 3% and preferably ≤ 90% and more preferably ≤ 80% based on the external height of the purification column. Regarding a divided wall column as purification column, the relative positions of feed point and the sampling point have a minor influence on the temper stability of the C₆₋₁₂ saturated aliphatic carboxylic acids.

Based on the above description regarding the distillation device and its operation, the person skilled in the art is able to design the distillation device and to determine the appropriate operation conditions depending on the nature of the C₆₋₁₂ saturated aliphatic carboxylic acid.

The above-mentioned process enables the preparation of C₆₋₁₂ saturated aliphatic carboxylic acids in a high purity with a content of ≥ 95 wt.-% of the C₆₋₁₂ saturated aliphatic carboxylic acid based on the distillate and having a very low APHA color number even after being tempered under inert gas conditions at a temperature of 225°C and a pressure of 0.1 MPa for 4 hours. Expressed in numbers, the purified distillate containing ≥ 95 wt.-% of the saturated aliphatic carboxylic acid based on the distillate has an APHA color number of 0 to 10 after being tempered under inert gas conditions at a temperature of 225°C and a pressure of 0.1 MPa for 4 hours. The content of the C₆₋₁₂ saturated aliphatic carboxylic acid is preferably ≥ 98 wt.-%, more preferably ≥ 99 wt.-%, particularly preferably ≥ 99.5 wt.-% and very particularly preferably ≥ 99.8 wt.-% based on the distillate. As typical by-products, formates, carboxylic acids with a lower number of carbon atoms, alcohols, ketones and water are mentioned. For the sake of clarity, it is mentioned that the APHA color number refers to the established and well-known Hazen scale according to DIN EN ISO 6271 and ASTM D1209, respectively.

According to the low APHA color number after the above-mentioned temper procedure, also the APHA color number of the freshly distilled, non-tempered C₆₋₁₂ saturated aliphatic carboxylic acids is very low and generally 0 to 10, preferably ≤ 5 and more preferably ≤ 3.

The above-mentioned temper procedure under which the C₆₋₁₂ saturated aliphatic carboxylic acid is tempered under inert gas conditions at a temperature of 225°C and a pressure of 0.1 MPa for 4 hours simulates under defined and reproducible conditions a thermal stress scenario and as such is a more or less trustworthy indication of the color stability during storage, when subjected to thermal stress and/or after their regular application in the manufacture of products.

In more detail, the so-called temper test is conducted as follows: 40.0 to 60.0 g of the C₆₋₁₂ saturated aliphatic carboxylic acid containing sample is placed in a 3-neck round-bottom flask, equipped with a magnetic stirrer, a condenser and a glass frit for bubbling inert gas (about 0.2 Nl/min, preferably argon 5.0) through the sample. At ambient temperature, the sample is sparged with inert gas for 30 min while stirring. The sparging is then reduced to a minimum and the flask lowered into a preheated oil bath kept at 225°C. After 4 hours the sparging rate is slightly increased and the heating bath removed. It is important that the sparging rate is high enough to prevent air from being sucked into the flask during cooling. When the flask has cooled down to ambient temperature, a sample is taken and the APHA color number is measured. The measurement can for instance be carried out in a Hach LICO^{®} 620 colorimeter using a 10 mL cuvette. The instrument is calibrated against distilled water in advance.

The APHA color number of the purified C₆₋₁₂ saturated aliphatic carboxylic acid distillate after being tempered by the temper test as describe above is preferably ≤ 8 and more preferably ≤ 5. Although an APHA color number of 0 can be achieved, it is frequently ≥ 1.

Since the discoloration of the C₆₋₁₂ saturated aliphatic carboxylic acid distillate obtained in step (c) during the temper test is mainly caused by the presence of small amounts of peroxides, the content of active oxygen in the non-tempered C₆₋₁₂ saturated aliphatic carboxylic acid distillate is a further indicator for its color stability. According to the invention, the purified distillate obtained in step (c) preferably has an active oxygen content of 0 to 100 wt.-ppm, more preferably of ≤ 50 wt.-ppm and particularly preferably of ≤ 10 wt.-ppm based on the distillate, and preferably ≥ 1 wt.-ppm.

As already mentioned before, each step of steps (a) to (c) can be performed continuously or discontinuously, whereas for step (a) also a semi-continuous operation is an additional option. It may be advantageous for lower production volumes to produce the C₆₋₁₂ saturated aliphatic carboxylic acids in a discontinuous or semi-continuous process since such discontinuous and semi-continuous processes are normally more flexible and easier to operate for small production volumes. On the other hand, a continuous process in which steps (a) to (c) are performed continuously has the advantage of being more efficient as soon as the process is started up and stably running. It is therefore a preferred option for higher capacities of > 1000 tons per year to prepare the C₆₋₁₂ saturated aliphatic carboxylic acids continuously. This particularly applies for saturated aliphatic carboxylic acids which are produced in large volumes, as for example for octanoic acid, 2-ethylhexanoic acid, nonanoic acid, 3,5,5-trimethylhexanoic acid, decanoic acid, 2-propylheptanoic acid and dodecanoic acid.

In a general embodiment for the continuous preparation of 2-ethylhexanoic acid, liquid 2-ethylhexanal, an aqueous solution of potassium hydroxide as selectivity improving additive and gaseous oxygen are continuously fed into a jet loop reactor, which is operated at a temperature in the range of 30 to 60°C and at an oxygen partial pressure in the range of 0.11 to 1 MPa abs. To remove the heat produced by the exothermal oxidation reaction, the reactor is externally cooled by an external heat exchanger. The reaction mixture obtained by the above-mentioned step (a) still contains non-converted 2-ethylhexanal in an amount of 0.1 to 2 mol-% with respect to 2-ethylhexanoic acid and molecular oxygen in a concentration of 20 to 500 wt.-ppm, depending on the oxygen partial pressure, the temperature and the conversion rate based on the liquid mixture. It is fed to a stripping tower and stripped at 40 to 60°C and about atmospheric pressure or under vacuum conditions with a stream of nitrogen in an amount of 0.5 to 4 mol nitrogen per mol molecular oxygen to obtain a molecular oxygen depleted mixture containing ≤ 10 wt.-ppm based on the liquid mixture. The molecular oxygen depleted mixture is then continuously fed into a distillation device operated at vacuum conditions containing a low boiler column for separating off the low boilers such as water, the remaining 2-ethylhexanal and other low-boiling by-products, and a purification column which is fed with the bottom stream of the low boiler column and in which the purified 2-ethylhexanoic acid is withdrawn as a side stream. Optionally, the two columns can also be combined into one single divided wall column. The 2-ethylhexanoic acid obtained by the above-mentioned distillation step (c) has a high purity and contains ≥ 99.5 wt.-% 2-ethylhexanoic acid. After being tempered under inert gas conditions at a temperature of 225°C and a pressure of 0.1 MPa for 4 hours, it only shows an APHA color number of ≤ 10.

In a general embodiment for the discontinuous preparation of 2-ethylhexanoic acid, 2-ethylhexanoic acid as diluting solvent and potassium hydroxide as selectivity improving additive are placed in a temperature controlled stirred vessel, and 2-ethylhexanal and molecular oxygen or a molecular oxygen containing gas mixture are simultaneously fed to the stirred vessel at a total pressure of 0.1 to 10 MPa abs during 0.1 to 5 hours while controlling the temperature at 25 to 60°C. After the desired liquid level in the stirred vessel is attained, the addition of 2-ethylhexanal is stopped and molecular oxygen replenished to hold the partial pressure and to bring the conversion to the desired value. After the desired conversion is reached, also the addition of molecular oxygen is stopped and replaced by the addition of nitrogen for stripping at 40 to 60°C and about atmospheric pressure or under vacuum conditions, while the stirring in the vessel is still continued. Typically, a nitrogen stream of 0.5 to 100 mol nitrogen per mol of dissolved molecular oxygen is fed over a time of 10 minutes to 2 hours to obtain a molecular oxygen depleted mixture containing ≤ 10 wt.-ppm based on the liquid mixture. The molecular oxygen depleted mixture is then either placed in a batch vacuum distillation column and, after having removed the low boilers, a fraction containing purified 2-ethylhexanoic acid obtained, or continuously fed to a continuously operated distillation device and purified 2-ethylhexanoic acid withdrawn therefrom. The purified 2-ethylhexanoic acid obtained has a high purity and contains ≥ 99.5 wt.-% 2-ethylhexanoic acid. After being tempered under inert gas conditions at a temperature of 225°C and a pressure of 0.1 MPa for 4 hours, it only shows an APHA color number of ≤ 10.

Beside the above-mentioned process, 2-ethylhexanoic acid having an APHA color number of 0 to 10 after being tempered under inert gas conditions at a temperature of 225°C and a pressure of 0.1 MPa for 4 hours was found. This was insofar astonishing as even 2-ethylhexanoic acid produced by methods described in the state of the art by processes other than oxidation processes, such as for example by dehydrogenating 2-ethylhexanol with NaOH to sodium 2-ethylhexanoate and subsequent acidification with H₂SO₄, do not result in a temper stable 2-ethylhexanoic acid fulfilling the above-mentioned temper test although no peroxides are expected to be formed in this process.

The process of the invention enables the preparation of saturated aliphatic carboxylic acids with 6 to 12 carbon atoms by oxidation of the corresponding aldehyde with oxygen in high yield and high purity, and particularly with a high color stability, so that even after a temper procedure under inert gas conditions at a temperature of 225°C and a pressure of 0.1 MPa for 4 hours the APHA color number remains very low. The C₆₋₁₂ saturated aliphatic carboxylic acids also have a very low content of active oxygen and consequently a very low content of peroxides such as peroxy acids, hydroperoxides and other peroxides. The process can also be easy operated and functions stably over long operating times and producing the saturated aliphatic carboxylic acids in a constant high quality. The C₆₋₁₂ saturated aliphatic carboxylic acids can be stored over a long time, thermally exposed or applied in the production of further products without or at least with a very low tendency of darkening.

Furthermore, the process enables the access to high purity and temper stable 2-ethylhexanoic acid.

### Examples

### Determination of active oxygen by iodometry

The content of active oxygen in the samples was determined by iodometry. The following gives a general description on how the determination was performed.

Approximately 5 g of the sample, weighed to the nearest 0.1 mg, are placed in a reaction vial, flushed with argon, and 40 ml of a 1:1 acetic acid / chloroform mixture added to dissolve the sample. The reaction vial is provided with a cooler and placed in a stirring heating block, that is already preheated to 80°C. A weak argon flow is passed through the cooler to prevent the ingress of air. After the temperature has equilibrated, 5.0 mL of a saturated potassium iodide solution (ca. 60.0 g potassium iodide dissolved in 100 mL deionized water) are added through the cooler and the mixture is boiled under reflux for 10 min. In the next step, 40.0 mL deionized water are added, and the sample solution is titrated with a 0.01 M thiosulfate solution while using a platinum electrode as end point indicator.

### Determination of molecular oxygen

The content of molecular oxygen in the samples was determined by a high precision and highly O₂ sensitive optical fluorescence sensor, which was suitable to measure molecular oxygen in carboxylic acids. In the examples, an optical sensor named FDO^{®} 925 from WTW was used. The measured data were cross-checked by additional measurements using a galvanic cell oxygen analyzer, which is also known as a Hersch cell.

### Determination of the APHA color number

The APHA color number of the samples was determined by a colorimeter which was in advance calibrated against distilled water. In the examples, a colorimeter named Lico^{®} 620 from Hach with a 10 mL cuvette was used.

### Description of the temper test

The so-called temper test simulates under defined and reproducible conditions a thermal stress scenario to indicate the color stability of the sample during storage, at thermal stress and/or after its regular application in the manufacture of products. The following gives a description on how the temper test was performed.

40.0 to 60.0 g of the sample is placed in a 3-neck round-bottom flask, equipped with a magnetic stirrer, a condenser and a glass frit for bubbling inert gas (about 0.2 NI/min argon 5.0) through the sample. At ambient temperature, the sample is sparged with inert gas for 30 min while stirring. The sparging is then reduced to a minimum, just to prevent air from being sucked into the flask and the flask lowered into a preheated oil bath kept at 225°C. After 4 hours the sparging rate is slightly increased and the heating bath removed. The slightly increased sparging rate has to be high enough to prevent air from being sucked into the flask during cooling. When the flask has cooled down to ambient temperature, a sample is taken and the APHA color number measured as described above.

### Example 1

### (Preparation of crude 2-ethylhexanoic acid)

Crude 2-ethylhexanoic acid was produced in accordance with step (a) of the invention. It was produced in a technical plant with a production capacity of around 3.75 tons 2-ethylhexanoic acid per hour by continuously oxidizing 2-ethylhexanal with pure oxygen at a temperature of 30-60°C and a pressure of 0.25 MPa abs and in the presence of 0.4 wt-% of potassium ions in the reaction mixture. The technical plant contained three reactors connected in series and the addition of the oxygen feed was divided between these three reactors. The 2-ethylhexanal and the potassium salt were added to the first reactor.

The crude 2-ethylhexanoic acid obtained at the exit of the third reactor contained 92.4 wt.-% 2-ethlyhexanoic acid and 281 wt.-ppm of active oxygen. The APHA color index was determined as > 1000. The content of molecular oxygen at the exit of the third reactor before decompression was 600 wt.-ppm. Even after cooling to ambient temperature and decompression to ambient pressure, the crude 2-ethylhexanoic acid still contained 210 wt.-ppm of molecular oxygen. The measured values are summarized in table 1.

### Example 2

### (Comparative example)

A sample of 1 kg of the crude 2-ethylhexanoic acid of example 1 was distilled in a batch distillation apparatus in pilot plant size containing a 2 m column packed with mesh rings. The column was operated at a top pressure of 1 kPa abs and 2-ethylhexanoic acid distilled off overhead and the fraction boiling at 102.5 ± 0.5°C was collected. The recovered 2-ethylhexanoic acid had a 2-ethylhexanoic acid content of 99.38 wt.-%, analyzed by gas chromatography, and contained 11 wt.-ppm active oxygen. Its APHA color number was 2. The measured values are summarized in table 1.

The distilled sample was then tempered under the conditions of the temper test as described above and the APHA color number measured. The APHA color number after the temper test was 40. This high value shows that the color stability of the 2-ethylhexanoic acid, which was processed according to the state of the art, was by far not sufficient.

### Example 3

### (Example according to the invention)

A further sample of 1 kg of the crude 2-ethylhexanoic acid of example 1 was first stripped at ambient temperature and pressure with high purity nitrogen (purity grade 5.0) until the content of the molecular oxygen was only 2 wt.-ppm. This procedure relates to step (b) of the invention. This sample was then distilled in the same batch distillation apparatus as used in example 2 containing a 2 m column packed with mesh rings in which special care was taken to minimize the leakage of air into the column by carefully greasing all the glass joints with high vacuum silicone grease. The column was operated at a top pressure of 1 kPa abs and 2-ethylhexanoic acid distilled off overhead and fraction boiling at 102.5 ± 0.5°C was collected. The recovered 2-ethylhexanoic acid had a 2-ethylhexanoic acid content of 99.99 wt.-%, analyzed by gas chromatography, and only contained 2 wt.-ppm active oxygen. Its APHA color number was 0 and the content of active oxygen was below the detection limit (< 1 ppm). The measured values are summarized in table 1.

The distilled sample was then tempered under the conditions of the temper test as described above and the APHA color number measured. The APHA color number after the temper test was only 7 which is in accordance with step (c). This low value shows that the inventive removal of molecular oxygen in step (b) to ≤ 10 wt.-ppm enables the preparation of a highly color stable 2-ethylhexanoic acid.

### Example 4

### (Effect of molecular oxygen on the APHA color number after the temper test)

A 500 ml sample of the purified 2-ethylhexanoic acid obtained in example 3 was filled in a 1-liter flask and immersed in a 40°C tempering bath without any precautions to exclude contact with air. Starting at time "0", a stream of 10 NL/h of air was bubbled through the sample via a glass frit. Samples were taken at regular intervals, analyzed for their content of active oxygen, molecular oxygen, and its APHA color numbers before and after the temper test. The results are summarized in table 2.

Since it took some time to transfer the sample of example 3 to the experimental set-up of example 4, the content of molecular oxygen raised from < 1 wt.-ppm to 14 wt.-ppm at the start of experiment 4 indicated by time "0". With ongoing time, the content of molecular oxygen increased up to 54 wt.-ppm after 5 hours (sample no. 3). With that increase, also the active oxygen content as well as the APHA color number after the temper procedure increased, whereas the APHA color number of the non-tempered sample remained at a value of 0. The increase of the active oxygen content indicates that part of the molecular oxygen was transformed into active oxygen since the molecular oxygen itself cannot be determined by the iodometry. After 5 hours (sample 3), the content of molecular oxygen remained at a constant value of 54 wt-ppm, which is be expected saturation concentration of molecular oxygen in 2-ethylhexanoic acid. Nevertheless, the content of active oxygen as well as the APHA color number after the temper test still increased with the time of bubbling air through the sample. This indicates that molecular oxygen is continuously transformed to active oxygen and caused together with the molecular oxygen a high APHA color number after the temper procedure.

### Example 5

### (Effect of a depletion of molecular oxygen on the APHA color number after the temper test)

Following example 4, the air sparging was stopped after 24 hours (i.e. after sample no. 5 was taken) and replaced by a sparging with 10 NUh nitrogen (purity grade 5.0) for 24 hours. After 24 hours of nitrogen sparging, a sample named sample no. 6 was taken and analyzed as in example 4.

The sparging with nitrogen had little to nearly no effect on the content of active oxygen, which only slightly decreased from 31 wt.-ppm in sample no. 5 to 28 wt.-ppm in sample no. 6. However, the amount of molecular oxygen was significantly decreased from 64 wt.-% in sample no. 5 to only 14 wt.-ppm in sample no. 6. This was a decrease in the order of a factor of 4. With that, the APHA color number after the temper test also decreased from 112 in sample no. 5 to 71 in sample no. 6. This demonstrates that the APHA color number after the temper test depends on both, on the content of molecular oxygen as well as on the content of active oxygen. With a significant reduction of the content of molecular oxygen in sample no. 6, the APHA color number after the temper test was also decreased.

**Table 1: Examples 1 to 3**

| Ex. | Description | APHA of sample | content of active oxygen [wt.-ppm] | content of molecular oxygen [wt.-ppm] | APHA after temper test |
|---|---|---|---|---|---|
| 1 | Crude 2EHA ^{#1} | > 1000 | 281 | n.m. ^{#2} | n.m. ^{#2} |
| 2 | Distilled 2EHA ^{#1} (according to the state of the art) | 2 | 11 | n.m. ^{#2} | 40 |
| 3 | Distilled 2EHA ^{#1} (according to the invention) | 0 | 2 | < 1 | 7 |

| | | | | | |
|---|---|---|---|---|---|
| ^{#1}: 2-ethylhexanoic acid ^{#2}: not measured | | | | | |

**Table 2: Examples 4 to 5**

| Ex. | Sample No. | Time [hours] | APHA of sample | content of active oxygen [wt.-ppm] | content of molecular oxygen [wt.-ppm] | APHA after temper test |
|---|---|---|---|---|---|---|
| 4 | 0 | 0 | 0 | 2 | 14 | 7 |
| | 1 | 1 | 0 | 11 | 21 | 31 |
| | 2 | 3 | 0 | 18 | 44 | 45 |
| | 3 | 5 | 0 | 24 | 54 | 85 |
| | 4 | 12 | 0 | 27 | 54 | 91 |
| | 5 | 24 | 0 | 31 | 54 | 112 |
| 5 | 6 | 24 | 0 | 28 | 14 | 71 |

## Claims

1. A process for the preparation of a saturated aliphatic carboxylic acid with 6 to 12 carbon atoms by oxidation of the corresponding aldehyde with molecular oxygen, which comprises
(a) converting the corresponding aldehyde with molecular oxygen at a temperature of 0 to 120°C and an oxygen partial pressure of 0.02 to 2 MPa to obtain a liquid mixture containing the saturated aliphatic carboxylic acid, ≤ 2 mol-% of the corresponding aldehyde with respect to the saturated aliphatic carboxylic acid, and molecular oxygen;
(b) removing molecular oxygen from the liquid mixture obtained in step (a) to a content of ≤ 10 wt.-ppm based on the liquid mixture; and
(c) distilling the mixture obtained in step (b) in a distillation device containing a purification column, and withdrawing therefrom a purified distillate containing ≥ 95 wt.-% of the saturated aliphatic carboxylic acid based on the distillate.

2. A process according to claim 1, wherein the saturated aliphatic carboxylic acid is 2-ethylhexanoic acid and the aldehyde is 2-ethylhexanal.

3. The process according to any of claims 1 to 2, wherein the content of molecular oxygen in the liquid mixture obtained in step (a) is > 10 wt.-ppm to ≤ 1 wt.-% based on the liquid mixture.

4. The process according to any of claims 1 to 3, wherein in step (b) molecular oxygen is removed by stripping with an inert gas.

5. A process according to claim 4, wherein the preparation of the saturated aliphatic carboxylic acid is performed continuously, and the stripping performed in a counter-current flow.

6. The process according to any of claims 4 to 5, wherein the inert gas has a content of molecular oxygen of ≤ 5 vol.-ppm.

7. The process according to any of claims 4 to 6, wherein in step (b) the removal of molecular oxygen is performed at a temperature of 0 to 150°C and a pressure of 0.0001 to 10 MPa abs.

8. The process according to any of claims 1 to 7, wherein in step (b) molecular oxygen is removed in step (b) to ≤ 2 wt.-ppm.

9. The process according to any of claims 1 to 8, wherein in step (c) the purification column is operated at a pressure of 0.1 to 99 kPa abs and a temperature of 0 to 170°C.

10. The process according to claim 9, wherein the amount of molecular oxygen withdrawn by the off-gas of the vacuum unit of the purification column is ≤ 2 times the amount of molecular oxygen fed to the distillation device by the mixture obtained in step (b).

11. The process according to claim 9, wherein the molar ratio n(Ar off-gas) / n(O₂ off-gas) in the off-gas of the vacuum unit of the purification column is 0 to 0.0222, wherein n(Ar off-gas) is the molecular amount of argon in the off-gas, corrected by the molecular amount of argon possibly fed to step (c) by the mixture obtained in step (b), and n(O₂ off-gas) is the molecular amount of molecular oxygen in the off-gas.

12. The process according to any of claims 1 to 11, wherein in step (c) the feed to the purification column, which is not a divided wall column, is added at a lower point than the purified distillate is withdrawn.

13. The process according to any of claims 1 to 12, wherein the purified distillate obtained in step (c) contains ≥ 99.5 wt.-% of the saturated aliphatic carboxylic acid.

14. A process according to any of claims 1 to 13, wherein the purified distillate has an APHA color number of 0 to 10 after being tempered under inert gas conditions at a temperature of 225°C and a pressure of 0.1 MPa for 4 hours.

15. The process according to any of claims 1 to 14, wherein the purified distillate obtained in step (c) has an active oxygen content of 0 to 100 wt.-ppm based on the distillate.

## Patentansprüche

1. Verfahren zur Herstellung einer gesättigten aliphatischen Carbonsäure mit 6 bis 12 Kohlenstoffatomen durch Oxidation des entsprechenden Aldehyds mit molekularem Sauerstoff, das Folgendes umfasst:
(a) Umwandeln des entsprechenden Aldehyds mit molekularem Sauerstoff bei einer Temperatur von 0 bis 120 °C und einem Sauerstoffpartialdruck von 0,02 bis 2 MPa unter Erhalt eines flüssigen Gemischs, das die gesättigte aliphatische Carbonsäure, ≤ 2 Mol-% des entsprechenden Aldehyds, bezogen auf die gesättigte aliphatische Carbonsäure, und molekularen Sauerstoff enthält;
(b) Entfernen von molekularem Sauerstoff aus dem in Schritt a) erhaltenen flüssigen Gemisch bis zu einem Gehalt von ≤ 10 Gew.-ppm, bezogen auf das flüssige Gemisch; und
(c) Destillieren des in Schritt (b) erhaltenen Gemischs in einer Destillationsvorrichtung mit einer Reinigungskolonne und Abziehen eines gereinigten Destillats, das ≥ 95 Gew.-% der gesättigten aliphatischen Carbonsäure, bezogen auf das Destillat, enthält, daraus.

2. Verfahren nach Anspruch 1, wobei es sich bei der gesättigten aliphatischen Carbonsäure um 2-Ethylhexansäure handelt und es sich bei dem Aldehyd um 2-Ethylhexanal handelt.

3. Verfahren nach einem der Ansprüche 1 bis 2, wobei der Gehalt an molekularem Sauerstoff in dem in Schritt (a) erhaltenen flüssigen Gemisch > 10 Gew.-ppm bis ≤ 1 Gew.-%, bezogen auf das flüssige Gemisch, beträgt.

4. Verfahren nach einem der Ansprüche 1 bis 3, wobei in Schritt (b) molekularer Sauerstoff durch Strippen mit einem Inertgas entfernt wird.

5. Verfahren nach Anspruch 4, wobei die Herstellung der gesättigten aliphatischen Carbonsäure kontinuierlich durchgeführt wird und das Strippen im Gegenstrom durchgeführt wird.

6. Verfahren nach einem der Ansprüche 4 bis 5, wobei das Inertgas einen Gehalt an molekularem Sauerstoff von ≤ 5 Vol.-ppm aufweist.

7. Verfahren nach einem der Ansprüche 4 bis 6, wobei in Schritt (b) die Entfernung von molekularem Sauerstoff bei einer Temperatur von 0 bis 150 °C und einem Druck von 0,0001 bis 10 MPa abs. durchgeführt wird.

8. Verfahren nach einem der Ansprüche 1 bis 7, wobei in Schritt (b) molekularer Sauerstoff bis ≤ 2 Gew.-ppm entfernt wird.

9. Verfahren nach einem der Ansprüche 1 bis 8, wobei in Schritt (c) die Reinigungskolonne bei einem Druck von 0,1 bis 99 kPa abs. und einer Temperatur von 0 bis 170 °C betrieben wird.

10. Verfahren nach Anspruch 9, wobei die durch das Abgas der Vakuumeinheit der Reinigungskolonne abgezogene Menge an molekularem Sauerstoff ≤ 2-mal die der Destillationsvorrichtung durch das in Schritt (b) erhaltene Gemisch zugeführte Menge an molekularem Sauerstoff ist.

11. Verfahren nach Anspruch 9, wobei das Molverhältnis n(Ar Abgas) / n(O₂ Abgas) in dem Abgas der Vakuumeinheit der Reinigungskolonne 0 bis 0,0222 beträgt, wobei n(Ar Abgas) die molekulare Menge an Argon in dem Abgas, korrigiert um die möglicherweise dem Schritt (c) durch das in Schritt (b) erhaltenen Gemisch zugeführte molekulare Menge an Argon, ist und n(O₂ Abgas) die molekulare Menge an molekularem Sauerstoff in dem Abgas ist.

12. Verfahren nach einem der Ansprüche 1 bis 11, wobei in Schritt (c) die Zufuhr zur Reinigungskolonne, bei der es sich nicht um eine Trennwandkolonne handelt, an einem tieferen Punkt als dem Abzugspunkt des gereinigten Destillats zugegeben wird.

13. Verfahren nach einem der Ansprüche 1 bis 12, wobei das in Schritt (c) erhaltene gereinigte Destillat ≥ 99,5 Gew.-% der gesättigten aliphatischen Carbonsäure enthält.

14. Verfahren nach einem der Ansprüche 1 bis 13, wobei das gereinigte Destillat nach Tempern unter Inertgasbedingungen bei einer Temperatur von 225 °C und einem Druck von 0,1 MPa über einen Zeitraum von 4 Stunden eine APHA-Farbzahl von 0 bis 10 aufweist.

15. Verfahren nach einem der Ansprüche 1 bis 14, wobei das in Schritt (c) erhaltene gereinigte Destillat einen Gehalt an aktivem Sauerstoff von 0 bis 100 Gew.-ppm, bezogen auf das Destillat, aufweist.

## Revendications

1. Procédé pour la préparation d'un acide carboxylique aliphatique saturé comportant 6 à 12 atomes de carbone par oxydation de l'aldéhyde correspondant avec de l'oxygène moléculaire, qui comprend
(a) la conversion de l'aldéhyde correspondant avec de l'oxygène moléculaire à une température de 0 à 120 °C et une pression partielle d'oxygène de 0,02 à 2 MPa pour obtenir un mélange liquide contenant l'acide carboxylique aliphatique saturé, ≤ 2 % en moles de l'aldéhyde correspondant par rapport à l'acide carboxylique aliphatique saturé, et l'oxygène moléculaire ;
(b) l'élimination de l'oxygène moléculaire du mélange liquide obtenu à l'étape (a) jusqu'à une teneur ≤ 10 ppm en poids sur la base du mélange liquide ; et
(c) la distillation du mélange obtenu à l'étape (b) dans un dispositif de distillation contenant une colonne de purification, et le soutirage de celle-ci d'un distillat purifié contenant ≥ 95 % en poids de l'acide carboxylique aliphatique saturé sur la base du distillat.

2. Procédé selon la revendication 1, dans lequel l'acide carboxylique aliphatique saturé est de l'acide 2-éthylhexanoïque et l'aldéhyde est le 2-éthylhexanal.

3. Procédé selon l'une quelconque des revendications 1 à 2, dans lequel la teneur en oxygène moléculaire dans le mélange liquide obtenu à l'étape (a) est > 10 ppm en poids à ≤ 1 % en poids sur la base du mélange liquide.

4. Procédé selon l'une quelconque des revendications 1 à 3, dans lequel à l'étape (b) de l'oxygène moléculaire est éliminé par strippage avec un gaz inerte.

5. Procédé selon la revendication 4, dans lequel la préparation de l'acide carboxylique aliphatique saturé est effectuée de manière continue, et le strippage effectué dans un flux à contre-courant.

6. Procédé selon l'une quelconque des revendications 4 à 5, dans lequel le gaz inerte a une teneur en oxygène moléculaire ≤ 5 ppm en volume.

7. Procédé selon l'une quelconque des revendications 4 à 6, dans lequel à l'étape (b) l'élimination de l'oxygène moléculaire est effectuée à une température de 0 à 150 °C et à une pression de 0,0001 à 10 MPa abs.

8. Procédé selon l'une quelconque des revendications 1 à 7, dans lequel à l'étape (b) de l'oxygène moléculaire est éliminé à l'étape (b) jusqu'à ≤ 2 ppm en poids.

9. Procédé selon l'une quelconque des revendications 1 à 8, dans lequel à l'étape (c) la colonne de purification est exploitée à une pression de 0,1 à 99 kPa abs et à une température de 0 à 170 °C.

10. Procédé selon la revendication 9, dans lequel la quantité d'oxygène moléculaire soutirée par l'effluent gazeux de l'unité de vide de la colonne de purification est ≤ 2 fois la quantité d'oxygène moléculaire alimentée au dispositif de distillation par le mélange obtenu à l'étape (b).

11. Procédé selon la revendication 9, dans lequel le rapport molaire n(Ar effluent gazeux) / n(O₂ effluent gazeux) dans l'effluent gazeux de l'unité de vide de la colonne de purification est de 0 à 0,0222, dans lequel n(Ar effluent gazeux) est la quantité moléculaire d'argon dans l'effluent gazeux, corrigé par la quantité moléculaire d'argon éventuellement alimenté à l'étape (c) par le mélange obtenu à l'étape (b), et n(O₂ effluent gazeux) est la quantité moléculaire d'oxygène moléculaire dans l'effluent gazeux.

12. Procédé selon l'une quelconque des revendications 1 à 11, dans lequel à l'étape (c), l'alimentation à la colonne de purification, qui n'est pas une colonne à paroi divisée, est ajoutée à un point inférieur au soutirage du distillat purifié.

13. Procédé selon l'une quelconque des revendications 1 à 12, dans lequel le distillat purifié obtenu à l'étape (c) contient ≥ 99,5 % en poids de l'acide carboxylique aliphatique saturé.

14. Procédé selon l'une quelconque des revendications 1 à 13, dans lequel le distillat purifié a un indice de couleur APHA de 0 à 10 après avoir été tempéré dans des conditions de gaz inerte à une température de 225 °C et à une pression de 0,1 MPa pendant 4 heures.

15. Procédé selon l'une quelconque des revendications 1 à 14, dans lequel le distillat purifié obtenu à l'étape (c) a une teneur en oxygène actif de 0 à 100 ppm en poids sur la base du distillat.
